# FASCICULE DE BREVET EUROPEEN

(11) **EP 3 282 958 B1**
(45) Date de publication et mention de la délivrance du brevet: **20.07.2022**
(21) Numéro de dépôt: 16727744.1
(22) Date de dépôt: 11.04.2016
(51) Int. Cl.: A61B 17/16, A61B 17/17, A61B 17/34, A61B 17/70, A61B 17/88

(54) **DISPOSITIF DE FUSION TRANSPÉDICULAIRE PERCUTANÉE**
VORRICHTUNG ZUR PERKUTANEN TRANSPEDIKULÄREN FUSION
DEVICE FOR PERCUTANEOUS TRANSPEDICULAR FUSION

(30) Priorité: 16.04.2015 FR 1553398
(43) Date de publication de la demande: 21.02.2018
(73) Titulaire: Clariance, 62217 Beaurains (FR)
(72) Inventeur: VIART, Guy, 62128 Saint Leger (FR); LEROY, Jean Yves, 62870 Campagne-les-Hesdin (FR); BILLON, Adrien, 59790 Rochin (FR); GANGI, Afshin, 77933 Lahr (DE); SCHULLER, Sébastien, 67400 Illkirch Graffenstaden (FR); STEIB, Jean Paul, 67100 Strasbourg (FR); MAZEL, Christian, 92350 Le Plessis Robinson (FR); VIRGAUX, Nicolas, 75018 Paris (FR)
(74) Mandataire: Vuillermoz, Bruno
(86) Numéro de dépôt international: PCT/FR2016/050833
(87) Numéro de publication internationale: WO 2016/207501

(56) Documents cités:
- US-A1- 2006 058 826
- US-A1- 2008 200 915
- US-A1- 2011 028 978
- US-A1- 2012 191 094
- US-B1- 6 558 386

## Description

La présente invention est relative à un dispositif de fusion transpédiculaire permettant la mise en place et l'injection d'un greffon autologue ou biologique dans un espace intersomatique Es préalablement aménagé entre deux vertèbres supérieure et inférieure Va, Vb d'un segment rachidien afin de réaliser une fusion intersomatique par un abord transpédiculaire totalement percutané.

On connait d'après la demande de brevet US2012/191094 un dispositif de forage pour former un canal osseux à profil incurvé via une canule droite préalablement fixée dans le corps d'une vertèbre, ledit dispositif comprenant une broche de guidage issue d'un kit de broche de guidage présentant à l'une de ses extrémités d'une part un profil courbe dont le rayon de courbure R est inférieur à 20 millimètres, et d'autre part une pointe effilée se trouvant dans une direction définie par un angle Y qui est inférieur à 90 degrés par rapport à l'axe longitudinal de ladite broche. On note que le profil de la broche de guidage permet de définir, après insertion de celle-ci dans le corps la vertèbre, deux points de contact a et b assurant le guidage de l'extrémité libre d'un foret articulé de manière à positionner ladite extrémité libre dans une direction sensiblement perpendiculaire à celle du plateau cortical de la vertèbre à percer.

On connait d'après la demande de brevet US2011/028978 un procédé et un dispositif permettant au chirurgien d'accéder à l'espace intervertébral pour restaurer une configuration tridimensionnelle plus normale de l'espace, avec ou sans fusion supplémentaire de deux vertèbres adjacentes.

On connait d'après la demande de brevet US2006/058826 un dispositif de cavitation tissulaire comprenant une canule, un tube d'insertion et un arbre de coupe, une partie de l'arbre de coupe étant engagée de manière coulissante dans la canule et une partie de la canule étant engagée de manière coulissante dans le tube d'insertion.

Depuis quelques années les chirurgiens intervenant dans le domaine des opérations rachidiennes sont très demandeurs de techniques simples, sures et rapides pour fusionner, par exemple, un segment rachidien.

Actuellement, les chirurgiens utilisent une technique dite de chirurgie ouverte entrainant une très large incision pour accéder à la zone à traiter et avoir une vue directe. Le traumatisme subit par le patient est important notamment lié à la dissection / désinsertion de tissus, la perte de sang et la longueur de l'incision. La chirurgie ouverte nécessite un temps opératoire court (car technique maitrisée) mais un temps d'hospitalisation long. Les coûts de santé liés sont très lourds.

Les chirurgiens sont à la recherche dans le domaine des chirurgies rachidiennes de techniques dites mini-invasives (MIS) pour permettre aux patients de récupérer plus rapidement après une opération chirurgicale. Ces techniques opératoires permettent de réduire la durée d'hospitalisation et de favoriser un retour rapide à une activité normale et donc de conduire à une réduction significative des coûts de santé.

Dans le domaine des chirurgies rachidiennes de techniques dites mini-invasives (MIS), on peut détailler les différentes voies d'abord et les chirurgies en mini accès (MAS) et percutanée.

La chirurgie mini accès (MAS) reprend le principe de la chirurgie mini-invasives (MIS) en utilisant de faibles incisions qui sont ouvertes au maximum avec une instrumentation spéciale. Le chirurgien à un contrôle visuel direct ou endoscopique sur la zone à traiter.

La chirurgie percutanée est plutôt que réaliser de petites incisions, les entrées dans le corps du patient sont des trous dans la peau au travers desquels des canules sont insérées. Les canules servent de voies sécurisées pour les instruments jusqu'à la zone à traiter. Toutes les insertions d'instruments et l'extraction de tissus se fait au travers de ces canules. Les dommages subis par le patient sont ainsi considérablement diminués mais aucun contrôle visuel direct est possible. L'utilisation de la fluoroscopie accrue entraine des risques radiologiques importants pour les chirurgiens et les patients.

On connait des techniques chirurgicales dites mini-invasives (MIS) utilisant des voies d'abord postérieur qui sont plus simples et plus répandues. Ces techniques consistent à mettre en place de fixations constituées de tiges de liaison immobilisées dans des vis poly-axiales transpédiculaires ancrées préalablement dans les corps vertébraux.

On constate que ces techniques par voie d'abord postérieur mini-invasif (MIS) ne permettent pas de réaliser une greffe postéro-latérale comme utilisée en chirurgie ouverte.

Généralement, la greffe se fait par la pose entre les corps vertébraux de deux vertèbres sus et sous jacente d'une cage intersomatique introduite par une voie d'abord postérieur complémentaire en mini accès (MAS).

Cette voie d'abord postérieur en mini accès (MAS) est mal aisée et fait perdre le bénéfice des techniques dites mini-invasives (MIS).

D'autre part, la difficulté d'accès par la voie d'abord postérieur rend difficile la préparation du site opératoire et l'avivement des plateaux vertébraux nécessaire pour une vascularisation de la greffe. Cette difficulté d'accès peut conduire à un taux d'échec de la fusion non négligeable.

On constate que tous ces inconvénients constituent un frein au développement de cette technique chirurgicale dite mini-invasive (MIS), malgré un potentiel très important.

Egalement, on remarque que la diminution importante des effets traumatiques des tissus existants par l'utilisation des techniques chirurgicale dite ouverte et la réduction au maximum des voies d'abord (réduction des perforations nécessaires pour atteindre le site à traiter) favorisent une récupération rapide des patients un temps d'hospitalisation réduit et un retour rapide à une activité normale.

L'objet de la présente invention consiste à proposer un dispositif de fusion transpédiculaire permettant la mise en place et l'injection d'un greffon autologue ou biologique dans un espace intersomatique préalablement aménagé entre deux vertèbres supérieure et inférieure Va, Vb d'un segment rachidien afin de réaliser une fusion intersomatique par abord transpédiculaire totalement percutané.

Le dispositif de fusion transpédiculaire suivant la présente invention est constitué d'au moins deux canules pédiculaires droites agencées respectivement soit sur la vertèbre supérieure Va, soit sur la vertèbre inférieure Vb soit sur les deux vertèbres Va, Vb du segment rachidien et servant de canaux de travail sécurisés et de positionneur de visées, d'au moins une broche guide à profil courbe qui est implantée dans le plateau Pi, Ps de la vertèbre correspondante Va, Vb au travers de la canule pédiculaire correspondante, d'un foret canulé flexible guidé autour de la broche guide correspondante et entrainé dans des mouvements de va et vient par un système d'entrainement canulé de manière à grignoter progressivement et de manière contrôlée des fenêtres F dans lesdits plateaux inférieur Pi et supérieur Ps de chaque vertèbre Va, Vb et de réduire en lambeaux les tissus nucléiques d'un disque intervertébral D pour la réalisation de l'espace intersomatique Es, d'un dispositif d'injection raccordé à l'une des canules pédiculaires droites pour l'injection d'un greffon dans l'espace intersomatique Es ainsi réalisé et d'obturateurs venant se visser dans les trous laissés libres après le retrait desdites canules pédiculaires droites.

Le dispositif de fusion transpédiculaire suivant la présente invention comporte un foret canulé flexible comprenant une tige canulée rigide se prolongeant par une zone longitudinale souple constituée par un câble de torsion creux et flexible solidaire à son extrémité libre d'une fraise canulée présentant des profils affûtés.

Le dispositif de fusion transpédiculaire suivant la présente invention comprend un foret canulé comportant à l'opposé de la fraise canulée des moyens de connexion agencés pour former une liaison avec le système d'entrainement canulé permettant d'entrainer dans des mouvements de va et vient et de rotation ledit foret canulé afin que la fraise canulée grignote progressivement et de manière contrôlée des fenêtres F dans lesdits plateaux inférieure Pi et supérieure Ps de chaque vertèbre Va, Vb et de réduire en lambeau les tissus nucléique.

Le dispositif de fusion transpédiculaire suivant la présente invention comprend une broche guide courbe comportant des moyens de connexion permettant son maintien, le contrôle de son positionnement et d'exercer sur cette dernière des mouvements de va-et-vient.

Le dispositif de fusion transpédiculaire suivant la présente invention comprend des obturateurs qui sont constitués de vis pédiculaires permettant l'agencement et la fixation de tiges de liaison d'un dispositif de fixation rachidien.

Le dispositif de fusion transpédiculaire suivant la présente invention comprend des obturateurs qui sont constitués d'une vis pédiculaire comportant un connecteur de liaison coopérant avec une vis de pression pour l'immobilisation en translation et en rotation d'une tige de liaison.

Des procédés de préparation de l'espace intersomatique, d'injection du greffon et de la mise en place des obturateurs selon le dispositif de fusion transpédiculaires sont décrits pour aider à la compréhension du dispositif mais non revendiqués.

Le procédé de préparation de l'espace intersomatique Es par abord transpédiculaire totalement percutané entre deux vertèbres Va, Vb d'un segment rachidien d'une colonne vertébrale suivant la présente invention consiste :
- A réaliser une visée transpédiculaire percutanée pour la mise en place et l'introduction dans les pédicules Pa et/ou Pb des vertèbres Va et/ou Vb de canules pédiculaires droites,
- A introduire dans les canules pédiculaires droites une broche guide courbe venant atteindre respectivement les plateaux vertébraux Pi, Ps des vertèbres Va, Vb,
- A enfiler sur la broche courbe un foret canulé connecté à un système d'entraînement de manière que l'extrémité distale flexible solidaire d'une fraise canulée dudit foret canulé vienne percer par des mouvements de va-et-vient les plateaux vertébraux Pi et Ps à l'endroit choisi et les tissus nucléiques du disque intervertébral D afin de réaliser un espace intersomatique Es,
- A nettoyer au moyen d'un dispositif de nettoyage introduit dans la canule pédiculaire droite correspondante, l'espace intersomatique Es par l'injection et l'aspiration de liquide physiologique afin d'évacuer un maximum de lambeaux de tissus nucléiques,
- Et à retirer la broche courbe et le dispositif de nettoyage de la canule pédiculaire droite correspondante.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste:
- A visser deux obturateurs en lieu et place des deux canules pédiculaires droites de la vertèbre inférieure Vb,
- A connecter un dispositif d'injection préalablement remplie de greffon autologue ou biologique sur l'une des canules pédiculaires droites de la vertèbre supérieure Va,
- A injecter le greffon au travers de l'une des canules pédiculaires droites afin de remplir l'espace intersomatique Es et jusqu'au débordement dudit greffon au travers de l'autre canule pédiculaire droite de la vertèbre supérieure Va,
- A visser un troisième obturateur en lieu et place de la canule pédiculaire droite de la vertèbre supérieure Va ne portant pas le dispositif d'injection,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A bloquer en translation et en rotation le premier dispositif de liaison dans les obturateurs,
- A déconnecter le dispositif d'injection et à retirer la dernière canule pédiculaire droite de la vertèbre Va pour la fixation d'un quatrième obturateur,
- Et à relier et bloquer les deux obturateurs restants par un deuxième dispositif de liaison.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste :
- A visser deux obturateurs en lieu et place des deux canules pédiculaires droites de la vertèbre supérieure Va,
- A connecter un dispositif d'injection préalablement remplie de greffon autologue ou biologique sur l'une des canules pédiculaires droites de la vertèbre inférieure Vb,
- A injecter le greffon au travers de l'une des canules pédiculaires droites afin de remplir l'espace intersomatique Es et jusqu'au débordement dudit greffon au travers de l'autre canule pédiculaire droite de la vertèbre inférieure Vb,
- A visser un troisième obturateur en lieu et place de la canule pédiculaire droite de la vertèbre inférieure Vb ne portant pas le dispositif d'injection,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A bloquer en translation et en rotation le premier dispositif de liaison dans les obturateurs,
- A déconnecter le dispositif d'injection et à retirer la dernière canule pédiculaire droite de la vertèbre inférieure Vb pour la fixation d'un quatrième obturateur,
- Et à relier et bloquer les deux obturateurs restants par un deuxième dispositif de liaison.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste :
- A visser deux obturateurs en lieu et place d'une canule pédiculaire droite de la vertèbre supérieure Va et d'une canule pédiculaire droite de la vertèbre inférieure Vb,
- A connecter un dispositif d'injection préalablement remplie de greffon autologue ou biologique sur la canule pédiculaire droite de la vertèbre supérieure Va,
- A injecter le greffon au travers de la canule pédiculaire droite de la vertèbre supérieure Va afin de remplir l'espace intersomatique Es et jusqu'au débordement dudit greffon au travers de la canule pédiculaire droite de la vertèbre inférieure Vb,
- A visser un troisième obturateur en lieu et place de la canule pédiculaire droite de la vertèbre inférieure Vb,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A bloquer en translation et en rotation le premier dispositif de liaison dans les obturateurs,
- A déconnecter le dispositif d'injection et à retirer la dernière canule pédiculaire droite de la vertèbre supérieure Va pour la fixation d'un quatrième obturateur,
- Et à relier et bloquer les deux obturateurs restants par un deuxième dispositif de liaison.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste :
- A visser deux obturateurs en lieu et place d'une canule pédiculaire droite de la vertèbre supérieure Va et d'une canule pédiculaire droite de la vertèbre inférieure Vb,
- A connecter un dispositif d'injection préalablement remplie de greffon autologue ou biologique sur la canule pédiculaire droite de la vertèbre inférieure Vb,
- A injecter le greffon au travers de la canule pédiculaire droite de la vertèbre inférieure Vb afin de remplir l'espace intersomatique Es et jusqu'au débordement dudit greffon au travers de la canule pédiculaire droite de la vertèbre supérieure Va,
- A visser un troisième obturateur en lieu et place de la canule pédiculaire droite de la vertèbre supérieure Va,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A bloquer en translation et en rotation le premier dispositif de liaison dans les obturateurs,
- A déconnecter le dispositif d'injection et à retirer la dernière canule pédiculaire droite de la vertèbre inférieure Vb pour la fixation d'un quatrième obturateur,
- Et à relier et bloquer les deux obturateurs restants par un deuxième dispositif de liaison.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste :
- A visser deux obturateurs dans les pédicules Pb de la vertèbre inférieure Vb,
- A connecter un dispositif d'injection préalablement remplie de greffon autologue ou biologique sur une canule pédiculaire droite de la vertèbre supérieure Va,
- A injecter le greffon au travers de l'une des canules pédiculaires droites de la vertèbre supérieure Va afin de remplir l'espace intersomatique Es et jusqu'au débordement dudit greffon au travers de l'autre canule pédiculaire droite de la vertèbre supérieure Va,
- A visser un troisième obturateur en lieu et place de la canule pédiculaire droite de la vertèbre supérieure Va ne portant pas le dispositif d'injection,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A bloquer en translation et en rotation le premier dispositif de liaison dans les obturateurs,
- A déconnecter le dispositif d'injection et à retirer la dernière canule pédiculaire droite de la vertèbre supérieure Va pour la fixation d'un quatrième obturateur,
- Et à relier et bloquer les deux obturateurs restants par un deuxième dispositif de liaison.

Le procédé d'injection d'un greffon par abord transpédiculaire totalement percutané dans un espace intersomatique Es ménagé entre les plateaux vertébraux Pi, Ps de vertèbres Va, Vb d'un segment rachidien d'une colonne vertébrale suivant la présente description consiste :
- A visser deux obturateurs dans les pédicules Pa de la vertèbre supérieure Va,
- A connecter un dispositif d'injection préalablement remplie de greffon autologue ou biologique sur une canule pédiculaire droite de la vertèbre inférieure Vb,
- A injecter le greffon au travers de l'une des canules pédiculaires droites de la vertèbre inférieure Vb afin de remplir l'espace intersomatique Es et jusqu'au débordement dudit greffon au travers de l'autre canule pédiculaire droite de la vertèbre inférieure Vb,
- A visser un troisième obturateur en lieu et place de la canule pédiculaire droite de la vertèbre inférieure Vb ne portant pas le dispositif d'injection,
- A relier les obturateurs se trouvant l'un au dessus de l'autre et solidaire des vertèbres Va, Vb par un premier dispositif de liaison,
- A poursuivre l'injection du greffon avec une mise sous pression afin de restaurer la hauteur discale entre les vertèbres Va, Vb,
- A déconnecter le dispositif d'injection et à retirer la dernière canule pédiculaire droite de la vertèbre inférieure Vb pour la fixation d'un quatrième obturateur,
- Et à relier et bloquer les deux obturateurs restants par un deuxième dispositif de liaison.

Le procédé de mise en place d'un obturateur par abord transpédiculaire totalement percutané dans les vertèbres Va, Vb d'un segment rachidien d'une colonne vertébrale suivant la présente description consiste :
- A Introduire un dispositif de broche sécurisée au travers de l'une des canule pédiculaire droite déjà en place jusqu'au niveau du mur antérieur de la vertèbre Va, Vb correspondante,
- A déployer la partie bifide de la broche sécurisée pour que cette dernière soit en appui dans l'os spongieux de la vertèbre Va, Vb,
- A retirer la canule pédiculaire droite correspondante de la vertèbre Va, Vb,
- A Visser une vis pédiculaire de l'obturateur dans le corps de la vertèbre Va, Vb au moyen du guidage de la broche sécurisée,
- A retirer la broche sécurisée au travers de la vis pédiculaire canulé avant la fin du vissage de cette dernière jusqu'à la profondeur désirée.

La description qui va suivre en regard des dessins annexés et donnés à titre d'exemples non limitatifs permettra de mieux comprendre l'invention, les caractéristiques qu'elle présente, et les avantages qu'elle est susceptible de procurer.

Les figures 1 à 4 sont des vues de profil illustrant un segment rachidien d'une colonne vertébrale dans lequel est aménagé un espace intersomatique par l'intermédiaire d'un dispositif de fusion transpédiculaire suivant la présente invention.

La figure 5 est une vue de dessous dans le plan coronal du disque intervertébral D illustrant le plateau inférieure de la vertèbre supérieure du segment rachidien grignoté au moyen du foret canulé flexible du dispositif de fusion transpédiculaire suivant la présente invention.

La Figure 6 est une vue montrant le foret canulé flexible du dispositif de fusion transpédiculaire suivant la présente invention.

La Figure 7 est une vue en perspective représentant un segment rachidien d'une colonne vertébrale coupé suivant un axe verticale permettant de montrer le profil de l'espace intersomatique réalisé avant l'introduction du greffon autologue ou biologique au moyen du dispositif de fusion transpédiculaire suivant la présente invention.

La Figure 8 est une vue en perspective illustrant un segment rachidien d'une colonne vertébrale coupé suivant un axe verticale permettant de montrer en vue de face l'espace intersomatique réalisé avant l'introduction du greffon autologue ou biologique au moyen du dispositif de fusion transpédiculaire suivant la présente invention.

Les Figures 9 à 12 sont des vues représentant les différentes étapes de préparation, d'injection du greffon autologue ou biologique dans l'espace intersomatique et de stabilisation d'un segment rachidien au moyen du dispositif de fusion transpédiculaire suivant la présente invention.

Les figures 13 à 22 sont des vues illustrant des variantes des différentes étapes de préparation, d'injection du greffon autologue ou biologique dans l'espace intersomatique et de stabilisation d'un segment rachidien au moyen du dispositif de fusion transpédiculaire suivant la présente invention

La figure 23 est une vue représentant un exemple d'obturateur implanté dans les pédicules des vertèbres du segment rachidien au moyen du dispositif de fusion transpédiculaire suivant la présente invention.

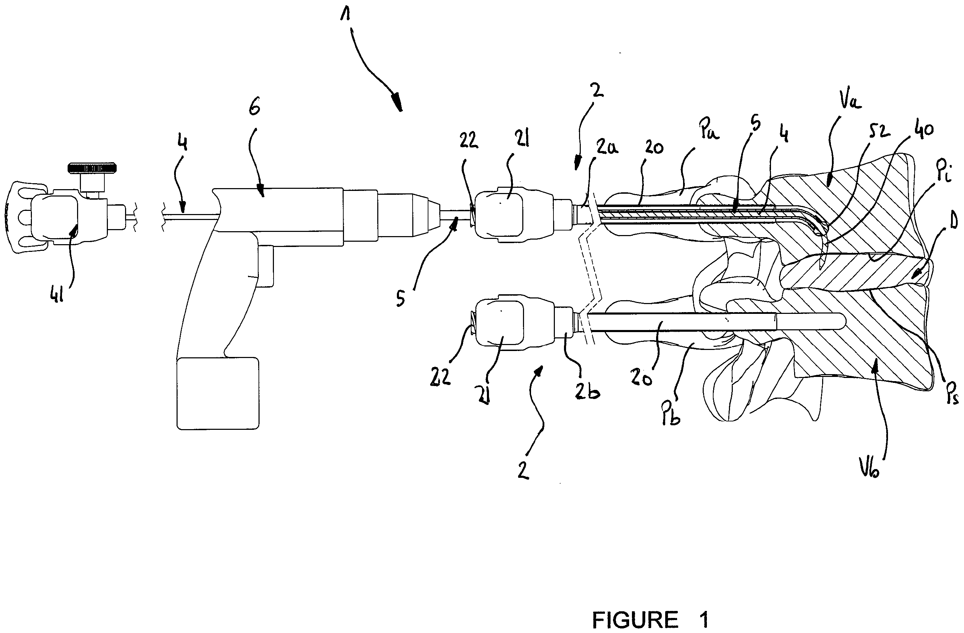

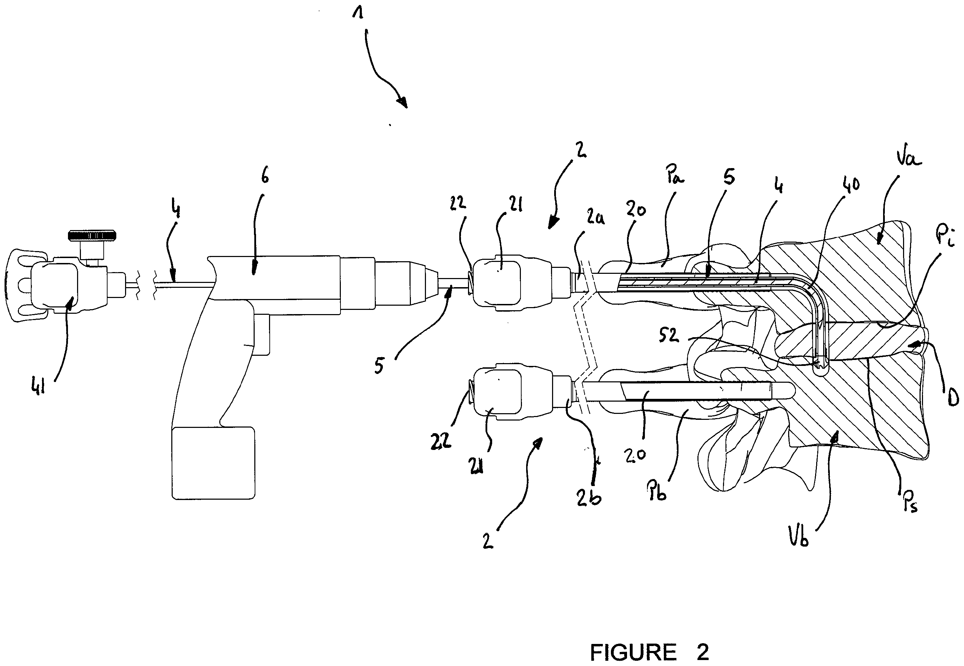

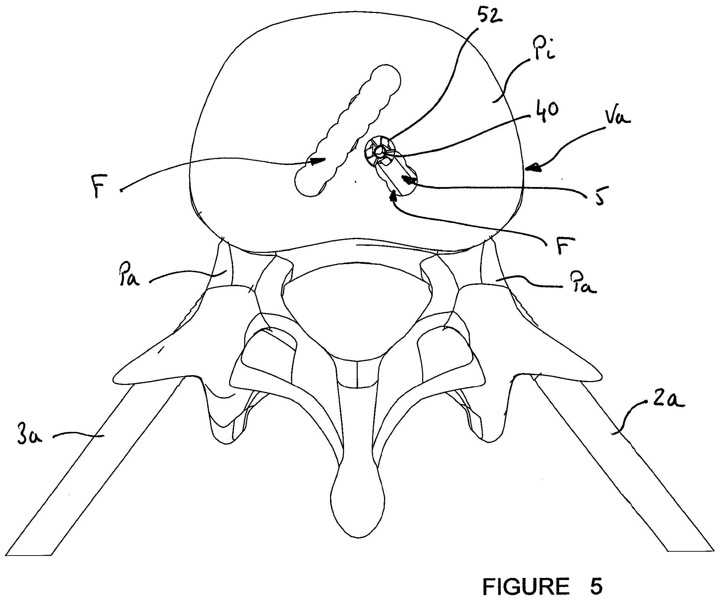

On a représenté en figures 1 à 5 un dispositif de fusion 1 suivant la présente invention comprenant deux canules pédiculaires droites 2, 3 par vertèbre Va, Vb servant de canal de travail sécurisé et de positionneur de visée.

Ainsi, pour la vertèbre Va, il sera vissé dans chaque pédicule Pa une canule pédiculaire droite 2a et 3a, tandis que pour la vertèbre Vb il sera vissé dans chaque pédicule Pb une canule pédiculaire droite 2b et 3b. Les canules pédiculaires droites 2a, 2b et/ou 3a, 3b lors des opérations de visée transpédiculaire, permettent de guider les différents instruments en direction des plateaux vertébraux et à l'endroit déterminé.

Chaque canule pédiculaire 2, 3 est constituée d'un tube 20, 30 solidaire à l'une de ses extrémités d'une tête de préhension 21, 31. La tête de préhension 21, 31 comporte en son milieu un moyen de connexion 22, 32 permettant l'immobilisation d'instrument.

Le dispositif de fusion 1 comprend au moins une broche guide 4 déformable élastiquement présentant en position déformée une extrémité à profil courbe pointue 40 assurant son implantation dans le plateau supérieur et/ou inférieur de la vertèbre correspondante Va, Vb. A l'opposé de l'extrémité à profil courbe 40 la broche guide 4 coopère avec un système de préhension 41 permettant au chirurgien de déplacer ladite broche.

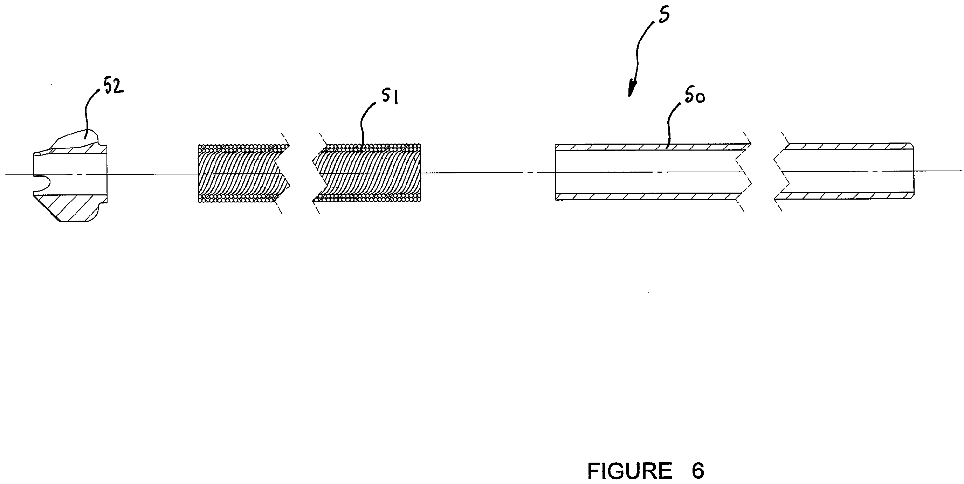

Le dispositif de fusion 1 comporte un système d'entrainement canulé 6 sur lequel est monté un foret canulé flexible 5 comprenant une tige canulée rigide 50 se prolongeant par une zone longitudinale souple constituée, par exemple, par un câble de torsion creux et flexible 51 solidaire à son extrémité libre d'une fraise canulée 52 présentant des profils affûtés (figure 6).

Le système d'entrainement canulé 6 permet d'entrainer dans des mouvements de va et vient et de rotation ledit foret canulé 5 afin que la fraise canulée 52 grignote progressivement et de manière contrôlée des fenêtres F dans lesdits plateaux supérieure et inférieure de chaque vertèbre Va, Vb et de réduire en lambeau les tissus nucléique du disque intervertébrale D.

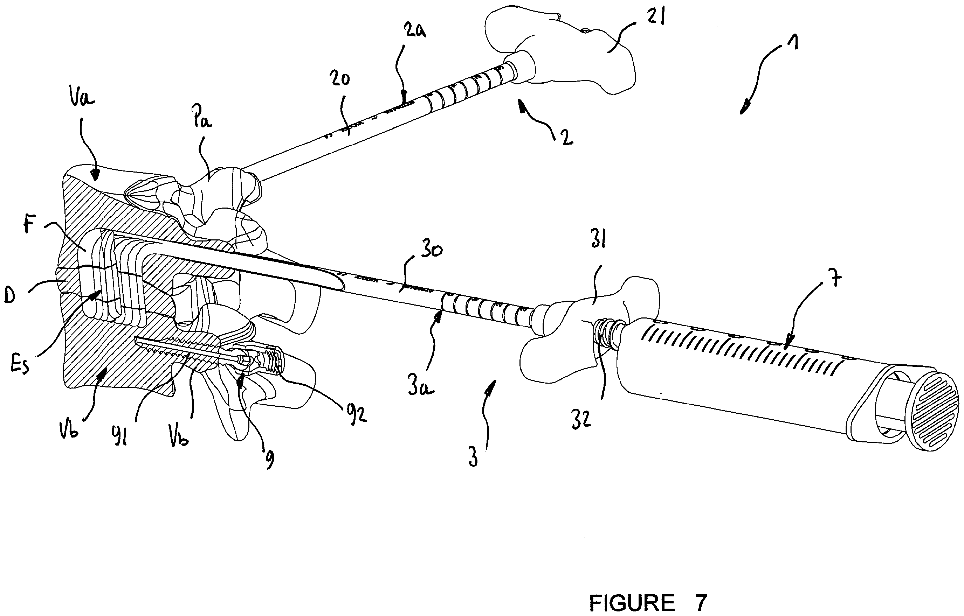

En figure 7 on a montré le dispositif de fusion 1 comportant un dispositif d'injection 7 qui est raccordé à l'une des canules pédiculaires 2a, 2b et 3a, 3b restées sur le site opératoire pour l'injection d'un greffon 8 dans l'espace intersomatique ainsi réalisé.

Le dispositif de fusion 1 comporte des obturateurs 9 venant se visser dans les trous laissés libres après le retrait des canules pédiculaires droites 2a, 2b et 3a, 3b.

Dans notre exemple de réalisation, les obturateurs 9 sont constitués de vis pédiculaires 90 reliées par des tiges de liaison 91 formant un dispositif de fixation rachidien connu en soi.

Le système d'entrainement 6 sur lequel est monté le foret canulé flexible 5 comporte des moyens de guidage permettant de laisser un libre passage à la broche guide courbe 4.

Après la réalisation de la visée transpédiculaire percutanée pratiqué par le chirurgiens, ce dernier prépare le site opératoire intersomatique au moyen du dispositif de fusion 1 suivant la présente invention en réalisant grâce à différents outils et un mode opératoire précis des fenêtres F dans le plateau vertébral inférieur Pi pour la vertèbre supérieure Va et supérieur Ps pour la vertèbre inférieure Vb. Ces outils permettent également une réduction en lambeaux des tissus nucléiques du disque intervertébral D se trouvant entre les vertèbres sus jacente Va et sous jacente Vb (figures 1 à 5).

Les canules pédiculaires droites 2a, 2b et 3a, 3b sont respectivement vissée dans le pédicule Pa, Pb correspondant de manière que la broche courbe 4 puisse atteindre les plateaux inférieur Pi et supérieur Ps à l'endroit déterminé par la position desdites canules pédiculaires droites 2a, 2b et 3a, 3b et percer lesdits plateaux vertébraux Pi et Ps par impaction.

Après avoir, dévissé les canules pédiculaires droites 2a, 2b et 3a, 3b correspondantes jusqu'au bord du mur postérieur de la vertèbre Va, Vb et de manière à éviter toute blessure des éléments neurologiques, le foret canulé 5 flexible en son extrémité distale est enfilé sur la broche courbe 4 qui va servir de guide.

Le foret canulé flexible 5 est connecté au système d'entrainement 6 comprenant un moteur assurant l'entrainement en rotation dudit foret pour la réalisation des fenêtres F.

Le chirurgien réalise le premier perçage ou fenêtre F des plateaux Pi, Ps et du disque intervertébral D en déplaçant le foret canulé flexible 5 autour de la broche guide courbe 4.

Les fenêtres F suivantes sont réalisées par un grignotage progressif des plateaux Pi, Ps et du disque intervertébral D.

Pour cela et après le premier perçage le chirurgien recul le système d'entrainement 6 solidaire du foret canulé flexible 5 afin de libérer la partie courbe de la broche guide 4.

Ensuite la broche guide 4 est déplacée pour venir se plaquer contre la paroi interne du premier perçage.

Lorsque la broche guide 4 est en place le système d'entrainement 6 est ramené pour que le foret canulé flexible 5 vienne élargir le premier perçage par grignotage des plateaux Pi, Ps et du disque intervertébral D afin de réaliser une seconde fenêtre F.

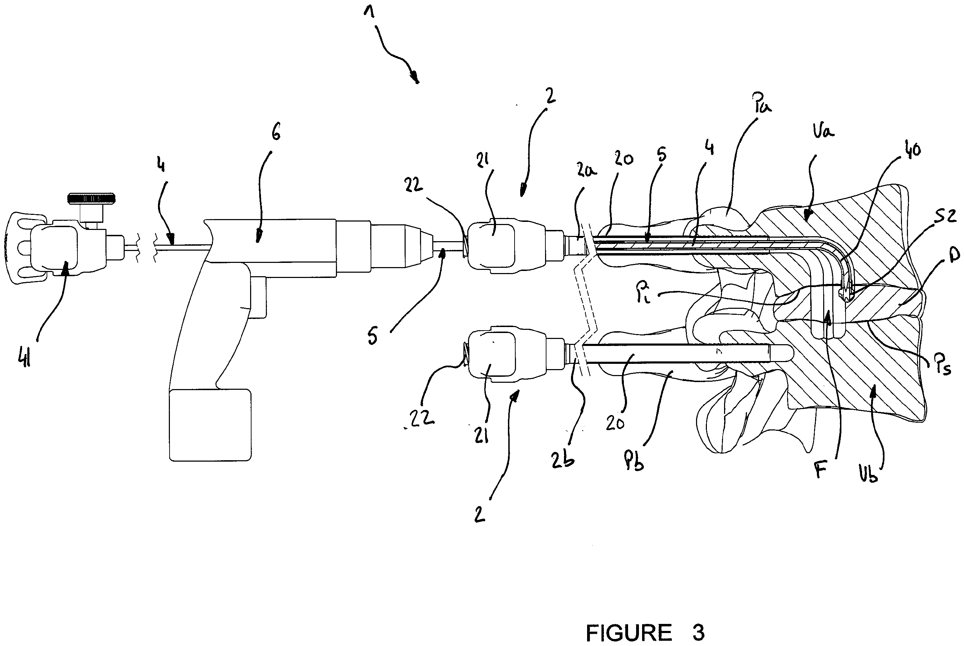

Pour cela le chirurgien déplace autant de fois qu'il est nécessaire le système d'entrainement 6 selon des mouvements de va-et-vient autour de la broche guide 4 de manière que le foret canulé flexible 5 réalise l'ensemble des fenêtres F par grignotage des plateaux vertébraux Pi, Ps correspondant (figures 3 à 5).

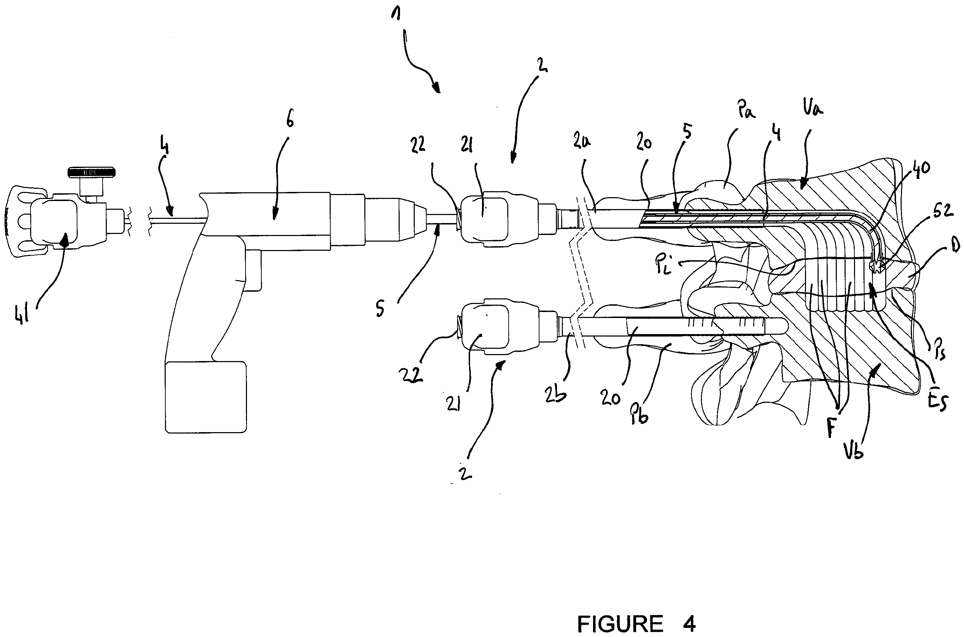

Le résultat obtenu par la répétition de ces opérations permet la réalisation d'un ensemble de fenêtres F permettant de réaliser un espace intra nucléique Es de large ouverture entre les vertèbres sus jacente Va et sous jacente Vb (figure 4).

Ces opérations pour creuser chacune de ces fenêtres F dans les plateaux vertébraux Pi, Ps sont réalisées au travers des canules pédiculaires droites 2a, 3a et/ou 2b, 3b solidaires respectivement des pédicules Pa, Pb des vertèbres adjacentes Va, Vb de la même manière que précédemment.

Ces opérations sont réalisées au moyen de la fraise canulée flexible 52 guidée par la broche courbe 4 introduite respectivement dans les canules pédiculaires droites 2a, 2b et/ou 3a, 3b, préalablement vissée dans les pédicules Pa et Pb lors des opérations de visée transpédiculaire, pour venir percer le plateau vertébral Pi et Ps à l'endroit choisi.

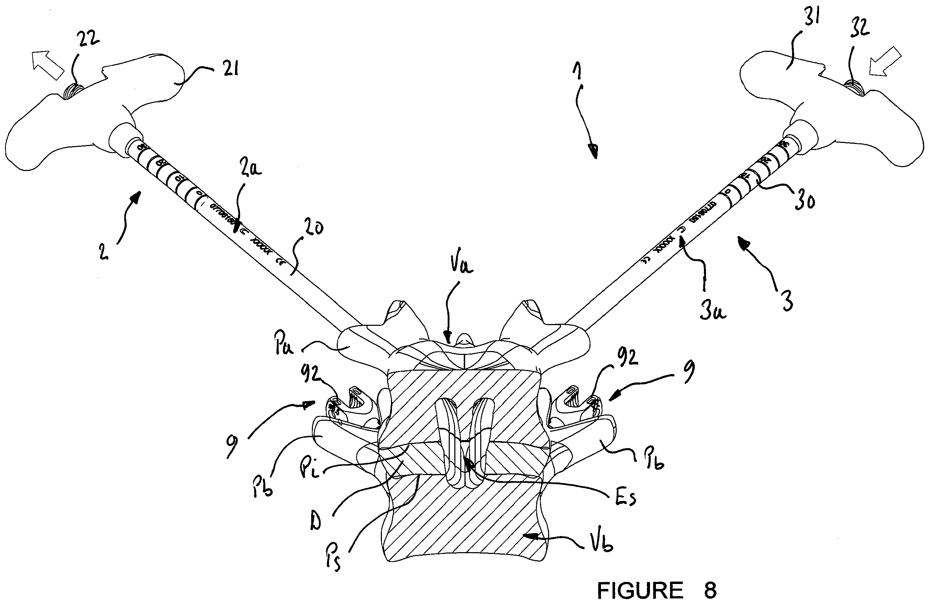

Le chirurgien procède ensuite au moyen des canules pédiculaires droites 2a, 3a et/ou 2b, 3b à un nettoyage du site opératoire à greffer par l'injection et l'aspiration de liquide physiologique afin d'évacuer un maximum de lambeaux de tissus nucléiques. A titre d'exemple le nettoyage du site opératoire à greffer peut être réalisé par l'introduction d'un tube souple au travers des canules venant déboucher dans l'espace intra nucléique Es afin d'éviter tout contact du liquide physiologique avec l'os spongieux des vertèbres Va, Vb (figure 8).

Avant l'injection du greffon, le chirurgien visse des obturateurs 9, tel que par exemple, des vis pédiculaires polyaxiales 90 dans les pédicules Pb de la vertèbre inférieure Vb (figures 7 et 8).

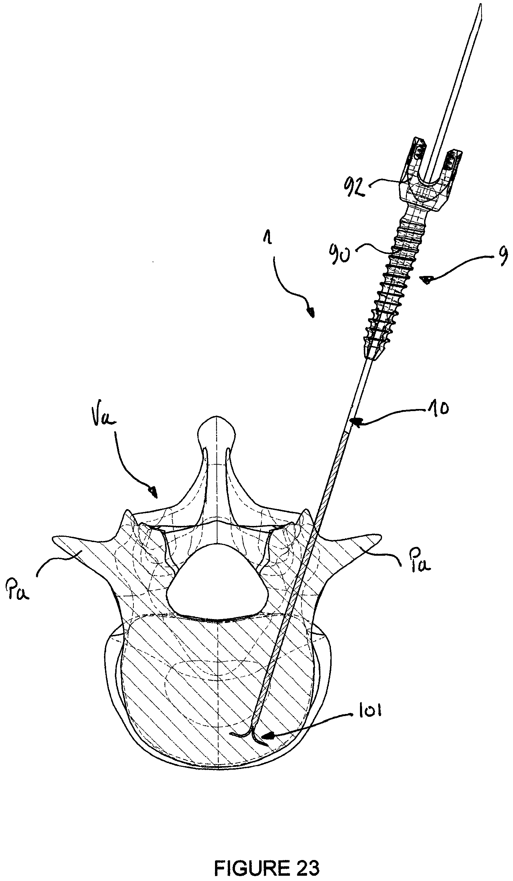

La mise en place des obturateurs 9 dans la vertèbre Vb est réalisée comme illustré en figure 23 et selon la procédure suivante :
- Introduction d'un dispositif de broche sécurisée 10 au travers de l'une des canule droite 2b, 3b déjà en place, jusqu'au niveau du mur antérieur ;
- Déploiement de la partie bifide 102 de la broche sécurisée 101 pour que cette dernière soit en appui dans l'os spongieux de la vertèbre Vb;
- Retrait de la canule droite 2b, 3b, correspondante de la vertèbre Vb;
- Vissage de la première vis pédiculaire 90 de l'obturateur 9 dans le corps de la vertèbre Vb au moyen du guidage de la broche sécurisée 101 ;
- Retrait de la broche sécurisée 101 au travers de la vis pédiculaire canulé 90 avant la fin du vissage de cette dernière ;
- Vissage complémentaire de la vis pédiculaire 90 jusqu'à la profondeur désirée.

Ces opérations sont reconduites pour la mise en place de la deuxième vis pédiculaire 90 formant l'obturateur 9 dans le la vertèbre inférieure Vb.

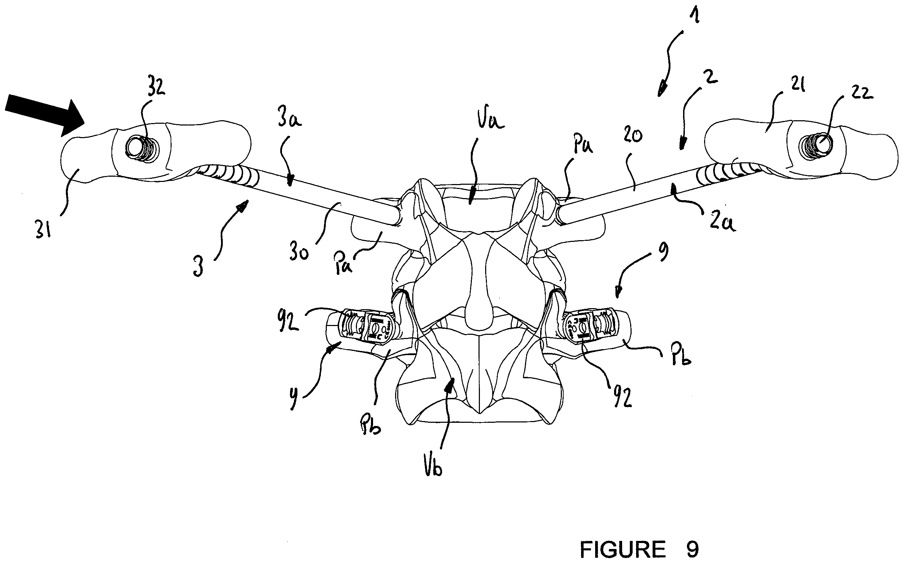

L'introduction de la greffe ou greffon 8 autologue ou biologique se réalise au moyen du dispositif d'injection 7 que le chirurgien connecte sur l'une des canules droites 2a, 3a implantées dans la vertèbre supérieure Va (figure 7 et 9).

La greffe autologue 8 peut être prélevée dans la crête iliaque et préparée avec des instruments spécifiques pour obtenir une pâte dont la granulométrie est compatible avec l'injection au travers d'une part du dispositif d'injection 7 et d'autre part de la canule droite 2a, 3a correspondante.

Dans le cas, de la greffe biologique 8, le produit est préparé à partir de différents composants dont la matrice de granulométrie est compatible avec une injection au travers d'une part du dispositif d'injection 7 et d'autre part de la canule droite 2a, 3a correspondante.

Le chirurgien effectue l'injection de la greffe 8 au travers, par exemple, de la canule droite 3a qui est préalablement raccordée au dispositif d'injection 7, jusqu'à ce que l'on puisse apercevoir l'écoulement du produit constituant la greffe 8 au travers de l'autre canule droite 2a afin de contrôler que l'espace intra nucléique Es soit parfaitement remplis.

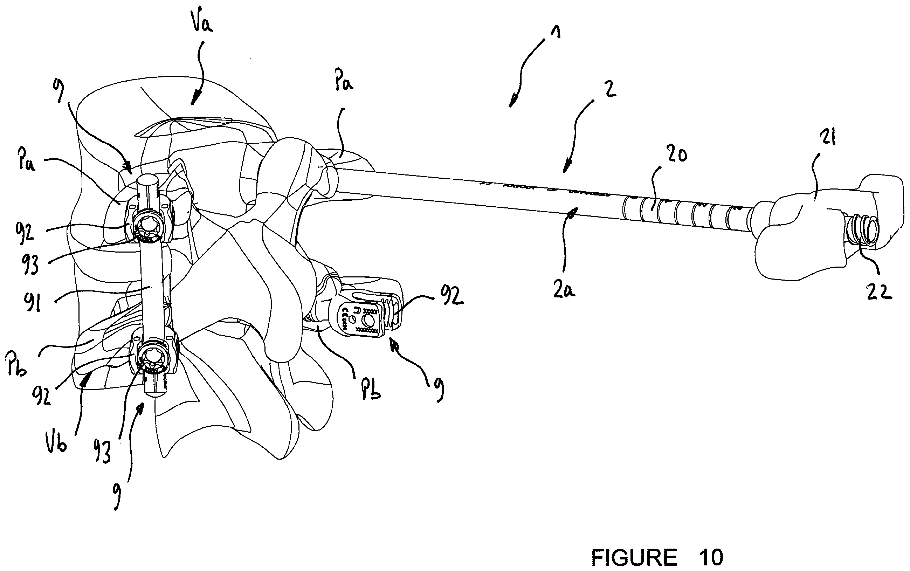

Le chirurgien procède à la fixation d'un troisième obturateur 9 constitué d'une vis pédiculaire 90 qui est vissée dans le pédicule de la vertèbre Va au moyen de la canule droite 3a et selon la même procédure que celle décrite précédemment pour la mise en place des vis pédiculaires 90 dans la vertèbre inférieure Vb (figure 10).

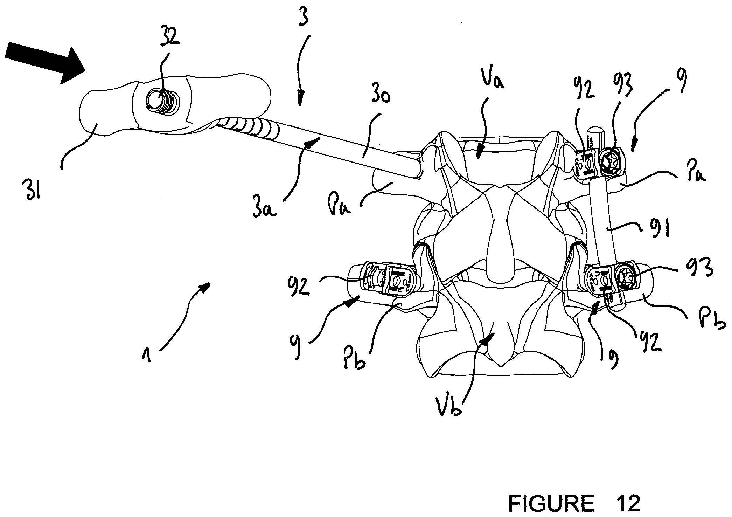

Ensuite, le chirurgien introduit une première tige de liaison 91 reliant les vis pédiculaires 90 se trouvant l'une au dessus de l'autre et solidaires des vertèbres Va et Vb. La première tige de liaison 91 est immobilisée dans des connecteurs 92 solidaires de chaque vis pédiculaire 90 par l'intermédiaire de vis de pression 93. Cette immobilisation est dans un premier temps limité pour garantir une liberté en translation de la tige de liaison 91 entre les vis pédiculaires 90 (figures 10 et 12).

Le chirurgien poursuit l'injection de la greffe 8 par l'intermédiaire du dispositif d'injection 7 connecté à la canule droite restante 2a ou 3a avec une mise sous pression afin de restaurer la hauteur discale entre les vertèbres Va et Vb du segment rachidien. La pression appliquée lors de l'injection du greffon 8 entre les vertèbres Va et Vb permet de remettre en tension l'annulus.

Cette opération est conduite sous contrôle radiographique en bloc opératoire et est arrêtée lorsque la hauteur discale parait satisfaisante (figures 10 et 12).

Dès que la hauteur discale entre les vertèbres Va et Vb parait satisfaisante, le chirurgien bloque définitivement la première tige de liaison 91 dans les connecteurs 92 des vis pédiculaires 90 par un serrage complémentaire des vis de pression 93.

Le dispositif d'injection 7 est déconnectée, de la canule droite 2a et le chirurgien procède à la mise en place d'un quatrième et dernier obturateur 9 constitué d'une vis pédiculaire 90 qui est vissée dans le pédicule de la vertèbre Va au moyen de ladite canule droite 2a et selon la même procédure que celle décrite précédemment pour la mise en place des vis pédiculaires 90 dans la vertèbre inférieure Vb .

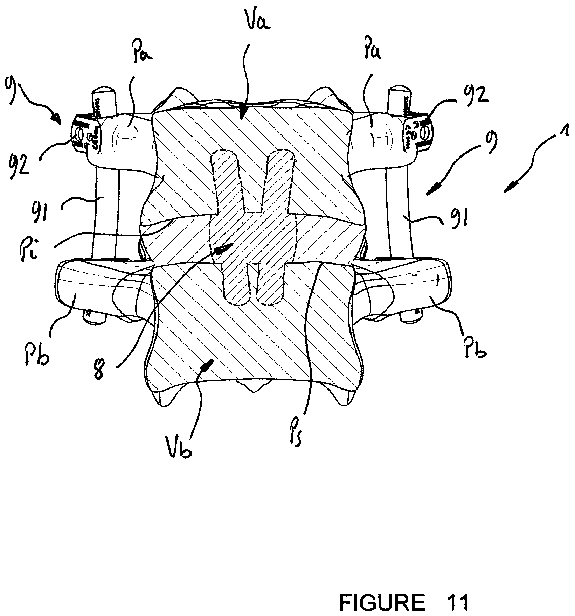

Ensuite, le chirurgien introduit une seconde tige de liaison 91 reliant les autres vis pédiculaires 90 se trouvant l'une au dessus de l'autre et solidaires des vertèbres Va et Vb. La seconde tige de liaison 91 est immobilisée dans des connecteurs 92 solidaires de chaque vis pédiculaire 90 par l'intermédiaire de vis de pression 93 (figure 11).

A ce stade, le chirurgien peut ajuster la lordose désirée par des manoeuvres de compression à l'aide d'un instrument spécifique et bloquer définitivement les tiges de liaison 91 dans les vis pédiculaires 90.

La fermeture des quatre incisions percutanés est réalisée de manière habituelle.

La greffe compactée grâce à son injection sous une certaine pression dans l'espace à greffer agit comme un étai intervertébral. En effet, la greffe maintenue compacte dans l'enveloppe constituée de l'annulus et des corps vertébraux procure un maintien efficace et dynamique favorable à la fusion.

De plus, la vascularisation du site greffé grâce au percement des plateaux vertébraux Pi, Ps permet d'obtenir des conditions très favorables (PH neutre et apports de nutriments et de composés bioactifs) pour une fusion rapide et de qualité. La sollicitation dynamique et la vascularisation abondante de la greffe permettent de réduire de manière conséquente le taux d'échec de la fusion.

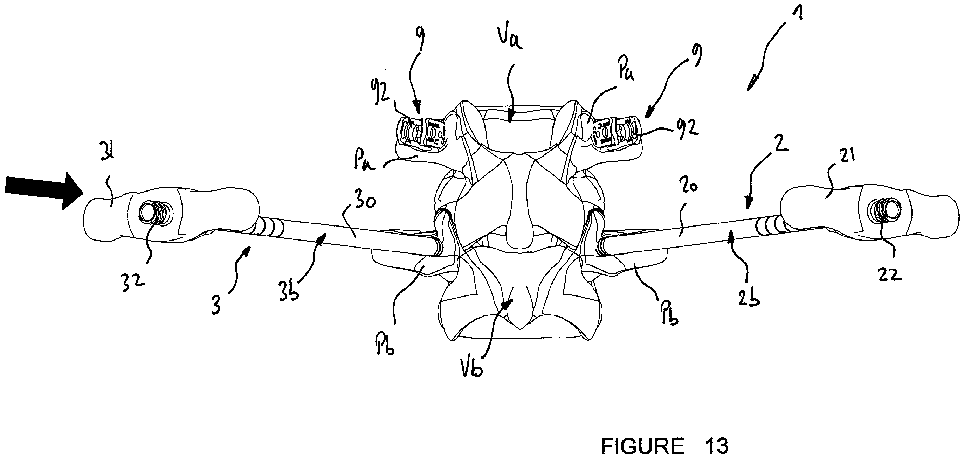

On a représenté en figures 13 à 22 différentes possibilités d'injection du greffon autologue ou biologique 8 par abord transpédiculaire totalement percutané dans l'espace intersomatique Es ménagé entre les plateaux vertébraux Pi, Ps des vertèbres Va, Vb du segment rachidien au moyen du dispositif de fusion transpédiculaire 1.

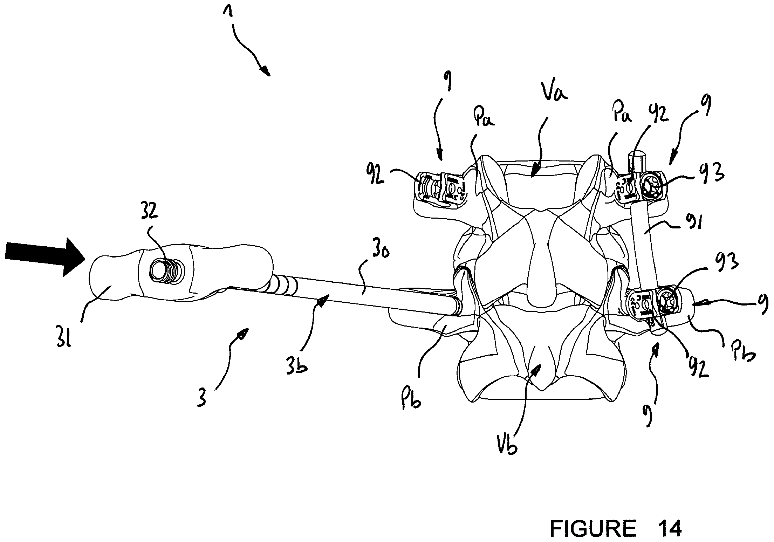

Les figures 13 et 14 montrent les étapes d'injection du greffon autologue ou biologique 8 suivantes :
- A visser deux obturateurs 9 en lieu et place des deux canules pédiculaires droites 2a, 3a de la vertèbre supérieure Va,
- A connecter un dispositif d'injection 7 préalablement remplie de greffon 8 autologue ou biologique sur l'une des canules pédiculaires droites 2b, 3b de la vertèbre inférieure Vb,
- A injecter le greffon 8 au travers de l'une des canules pédiculaires droites 2b, 3b afin de remplir l'espace intersomatique Es et jusqu'au débordement dudit greffon au travers de l'autre canule pédiculaire droite de la vertèbre inférieure Vb,
- A visser un troisième obturateur 9 en lieu et place de la canule pédiculaire droite de la vertèbre inférieure Vb ne portant pas le dispositif d'injection 7,
- A relier les obturateurs 9 se trouvant l'un au dessus de l'autre et solidaire des vertèbres Va, Vb par un premier dispositif de liaison,
- A poursuivre l'injection du greffon 8 avec une mise sous pression afin de restaurer la hauteur discale entre les vertèbres Va, Vb,
- A bloquer en translation et en rotation le premier dispositif de liaison dans les obturateurs 9,
- A déconnecter le dispositif d'injection 7 et à retirer la dernière canule pédiculaire droite de la vertèbre inférieure Vb pour la fixation d'un quatrième obturateur 9,
- Et à relier et bloquer les deux obturateurs 9 restants par un deuxième dispositif de liaison.

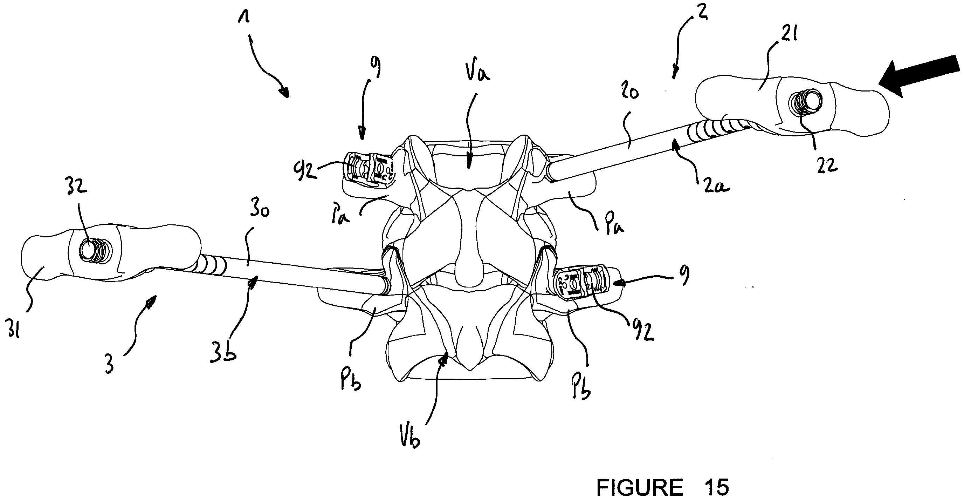

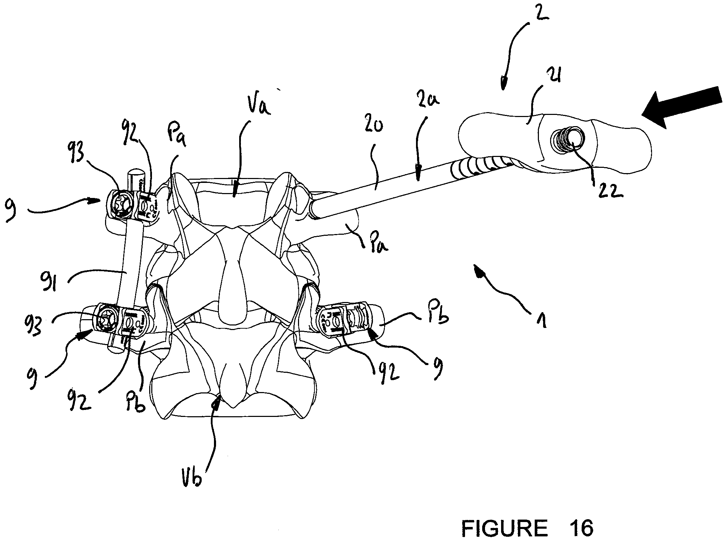

Les figures 15 et 16 montrent les étapes d'injection du greffon autologue ou biologique 8 suivantes :
- A visser deux obturateurs 9 en lieu et place d'une canule pédiculaire droite 2a, 3a de la vertèbre supérieure Va et d'une canule pédiculaire droite 2b, 3b de la vertèbre inférieure Vb,
- A connecter un dispositif d'injection 7 préalablement remplie de greffon 8 autologue ou biologique sur la canule pédiculaire droite 2a, 3a de la vertèbre supérieure Va,
- A injecter le greffon 8 au travers de la canule pédiculaire droite 2a, 3a de la vertèbre supérieure Va afin de remplir l'espace intersomatique Es et jusqu'au débordement dudit greffon au travers de la canule pédiculaire droite 2b, 3b de la vertèbre inférieure Vb,
- A visser un troisième obturateur 9 en lieu et place de la canule pédiculaire droite 2b, 3b de la vertèbre inférieure Vb,
- A relier les obturateurs 9 se trouvant l'un au dessus de l'autre et solidaire des vertèbres Va, Vb par un premier dispositif de liaison,
- A poursuivre l'injection du greffon 8 avec une mise sous pression afin de restaurer la hauteur discale entre les vertèbres Va, Vb,
- A bloquer en translation et en rotation le premier dispositif de liaison dans les obturateurs 9,
- A déconnecter le dispositif d'injection (7) et à retirer la dernière canule pédiculaire droite de la vertèbre supérieure Va pour la fixation d'un quatrième obturateur (9),
- Et à relier et bloquer les deux obturateurs (9) restants par un deuxième dispositif de liaison.

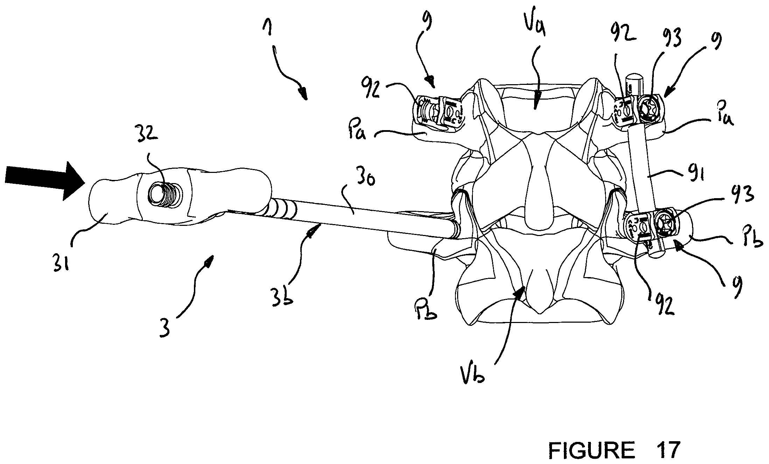

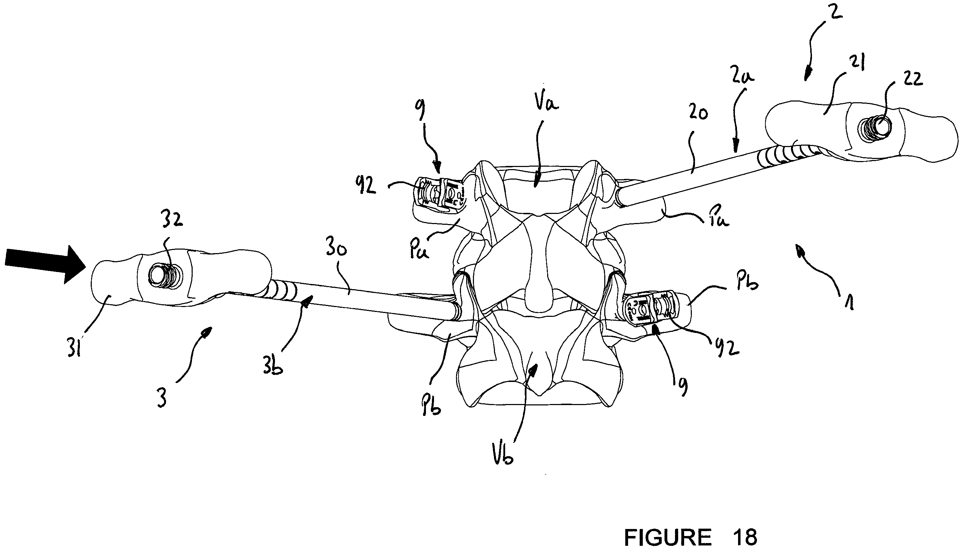

Les figures 17 et 18 représentent les étapes d'injection du greffon autologue ou biologique 8 suivantes :
- A visser deux obturateurs 9 en lieu et place d'une canule pédiculaire droite 2a, 3a de la vertèbre supérieure Va et d'une canule pédiculaire droite 2b, 3b de la vertèbre inférieure Vb,
- A connecter un dispositif d'injection 7 préalablement remplie de greffon 8 autologue ou biologique sur la canule pédiculaire droite 2b, 3b de la vertèbre inférieure Vb,
- A injecter le greffon 8 au travers de la canule pédiculaire droite 2b, 3b de la vertèbre inférieure Vb afin de remplir l'espace intersomatique Es et jusqu'au débordement dudit greffon au travers de la canule pédiculaire droite 2a, 3a de la vertèbre supérieure Va,
- A visser un troisième obturateur 9 en lieu et place de la canule pédiculaire droite 2a, 3a de la vertèbre supérieure Va,
- A relier les obturateurs 9 se trouvant l'un au dessus de l'autre et solidaire des vertèbres Va, Vb par un premier dispositif de liaison,
- A poursuivre l'injection du greffon 8 avec une mise sous pression afin de restaurer la hauteur discale entre les vertèbres Va, Vb,
- A bloquer en translation et en rotation le premier dispositif de liaison dans les obturateurs 9,
- A déconnecter le dispositif d'injection 7 et à retirer la dernière canule pédiculaire droite de la vertèbre inférieure Vb pour la fixation d'un quatrième obturateur 9,
- Et à relier et bloquer les deux obturateurs 9 restants par un deuxième dispositif de liaison.

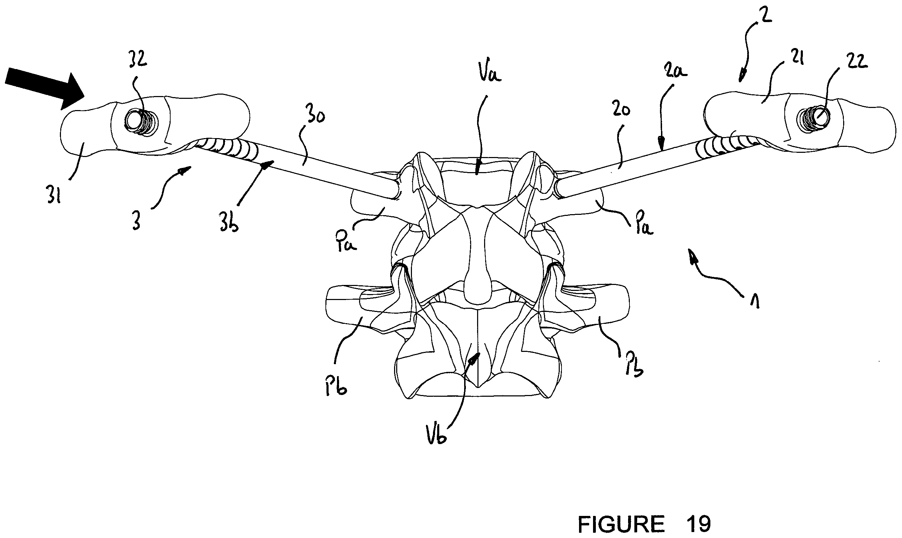

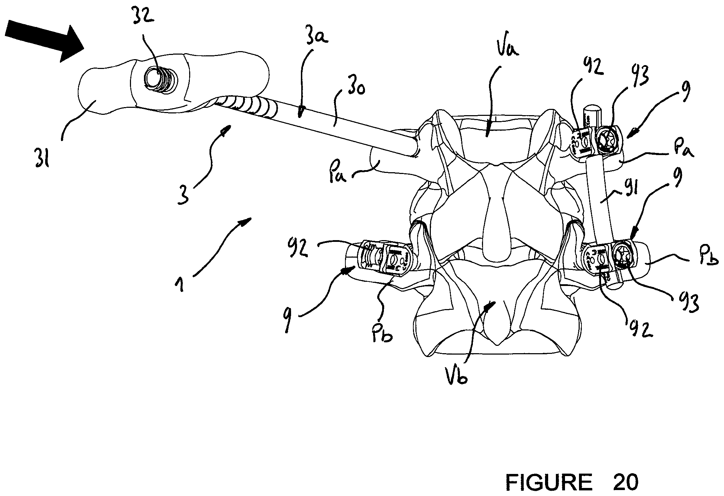

Les figures 19 et 20 illustrent les étapes d'injection du greffon autologue ou biologique 8 suivantes :
- A visser deux obturateurs 9 dans les pédicules Pb de la vertèbre inférieure Vb,
- A connecter un dispositif d'injection 7 préalablement remplie de greffon 8 autologue ou biologique sur une canule pédiculaire droite 2a, 3a de la vertèbre supérieure Va,
- A injecter le greffon 8 au travers de l'une des canules pédiculaires droites 2a, 3a de la vertèbre supérieure Va afin de remplir l'espace intersomatique Es et jusqu'au débordement dudit greffon au travers de l'autre canule pédiculaire droite 2a, 3a de la vertèbre supérieure Va,
- A visser un troisième obturateur 9 en lieu et place de la canule pédiculaire droite 2a, 3a de la vertèbre supérieure Va ne portant pas le dispositif d'injection 7,
- A relier les obturateurs 9 se trouvant l'un au dessus de l'autre et solidaire des vertèbres Va, Vb par un premier dispositif de liaison,
- A poursuivre l'injection du greffon 8 avec une mise sous pression afin de restaurer la hauteur discale entre les vertèbres Va, Vb,
- A bloquer en translation et en rotation le premier dispositif de liaison dans les obturateurs 9,
- A déconnecter le dispositif d'injection 7 et à retirer la dernière canule pédiculaire droite de la vertèbre supérieure Va pour la fixation d'un quatrième obturateur 9,
- Et à relier et bloquer les deux obturateurs 9 restants par un deuxième dispositif de liaison.

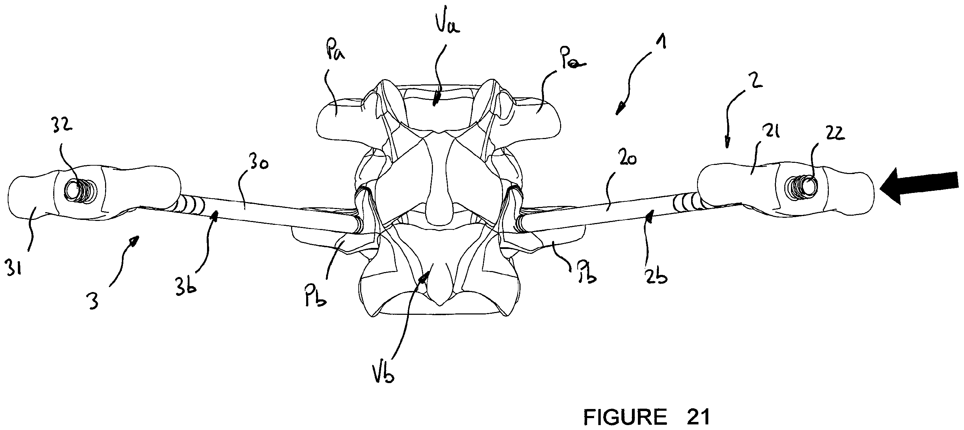

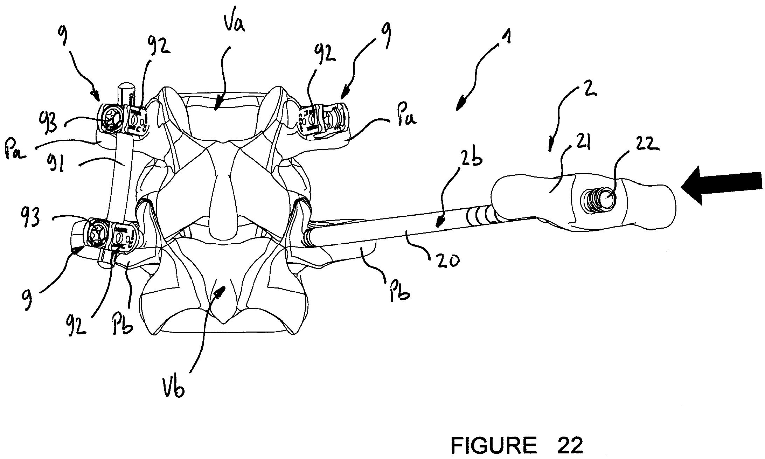

Les figures 21 et 22 montrent les étapes d'injection du greffon autologue ou biologique 8 suivantes :
- A visser deux obturateurs 9 dans les pédicules Pa de la vertèbre supérieure Va,
- A connecter un dispositif d'injection 7 préalablement remplie de greffon 8 autologue ou biologique sur une canule pédiculaire droite 2**b**, 3**b** de la vertèbre inférieure Vb,
- A injecter le greffon 8 au travers de l'une des canules pédiculaires droites 2b, 3**b** de la vertèbre inférieure Vb afin de remplir l'espace intersomatique Es et jusqu'au débordement dudit greffon au travers de l'autre canule pédiculaire droite 2**b**, 3**b** de la vertèbre inférieure Vb,
- A visser un troisième obturateur 9 en lieu et place de la canule pédiculaire droite 2**b**, 3**b** de la vertèbre inférieure Vb ne portant pas le dispositif d'injection 7,
- A relier les obturateurs 9 se trouvant l'un au dessus de l'autre et solidaire des vertèbres Va, Vb par un premier dispositif de liaison,
- A poursuivre l'injection du greffon 8 avec une mise sous pression afin de restaurer la hauteur discale entre les vertèbres Va, Vb,
- A bloquer en translation et en rotation le premier dispositif de liaison dans les obturateurs 9,
- A déconnecter le dispositif d'injection 7 et à retirer la dernière canule pédiculaire droite de la vertèbre inférieure Vb pour la fixation d'un quatrième obturateur 9,
- Et à relier et bloquer les deux obturateurs 9 restants par un deuxième dispositif de liaison.

## Revendications

1. Dispositif permettant la mise en place et l'injection d'un greffon (8) autologue ou biologique dans un espace intersomatique Es préalablement aménagé entre deux vertèbres supérieure et inférieure Va, Vb d'un segment rachidien afin de réaliser une fusion intersomatique par abord transpédiculaire totalement percutané, constitué d'au moins deux canules pédiculaires droites (2**a**, 3**a** ; 2**b**, 3**b**) agencées respectivement soit sur la vertèbre supérieure Va, soit sur la vertèbre inférieure Vb soit sur les deux vertèbres Va, Vb du segment rachidien, d'au moins une broche guide (4) à profil courbe qui est implantée dans le plateau Pi, Ps de la vertèbre correspondante Va, Vb au travers de la canule pédiculaire correspondante, d'un foret canulé flexible (5), **caractérisé en ce que** le forêt est guidé autour de la broche guide (4) correspondante et entrainé dans des mouvements de va et vient par un système d'entrainement canulé (6) de manière à grignoter progressivement et de manière contrôlée des fenêtres F dans lesdits plateaux inférieur Pi et supérieur Ps de chaque vertèbre Va, Vb et de réduire en lambeau les tissus nucléique d'un disque intervertébral D pour la réalisation de l'espace intersomatique Es, d'un dispositif d'injection (7) raccordée à l'une des canules pédiculaires droites (2**a**, 3**a** ; 2**b**, 3**b**) pour l'injection du greffon (8) dans l'espace intersomatique Es ainsi réalisé et d'obturateurs (9) venant se visser dans les trous laissés libres après le retrait desdites canules pédiculaires droites.

2. Dispositif suivant la revendication 1, **caractérisé en ce que** le foret canulé flexible (5) comprenant une tige canulée rigide (50) se prolongeant par une zone longitudinale souple constituée par un câble de torsion creux et flexible (51) solidaire à son extrémité libre d'une fraise canulée (52) présentant des profils affûtés.

3. Dispositif suivant la revendication 2, **caractérisé en ce que** à l'opposé de la fraise canulée (52) la tige rigide (50) du foret canulé (5) comporte des moyens de connexion agencés pour former une liaison avec le système d'entrainement canulé (6) permettant d'entrainer dans des mouvements de va et vient et de rotation ledit foret canulé (5) afin que la fraise canulée (52) grignote progressivement et de manière contrôlée des fenêtres F dans lesdits plateaux inférieure Pi et supérieure Ps de chaque vertèbre Va, Vb et de réduire en lambeau les tissus nucléique.

4. Dispositif suivant la revendication 1, **caractérisé en ce que** la broche guide courbe (4) comporte des moyens de connexion permettant son maintien, le contrôle de son positionnement et d'exercer sur cette dernière des mouvements de va-et-vient.

5. Dispositif suivant la revendication 1, **caractérisé en ce que** les obturateurs (9) sont des vis pédiculaires (90) d'un dispositif de fixation rachidien permettant la mise en place et la fixation de tiges de liaison (91).

6. Dispositif suivant la revendication 5, **caractérisé en ce que** chaque vis pédiculaire (90) comporte un connecteur de liaison (92) coopérant avec une vis de pression (93) pour l'immobilisation en translation et en rotation d'une tige de liaison (91).

## Patentansprüche

1. Vorrichtung, die das Anbringen und das Injizieren eines autologen oder biologischen Transplantats (6) in einem Zwischenwirbelraum Es erlaubt, der zuvor zwischen einem oberen und einem unteren Wirbel Va, Vb eines Wirbelsegments eingerichtet wird, um eine Zwischenwirbelraumfusion durch vollständig perkutanen transpedikulären Zugang herzustellen, der aus mindestens zwei geraden Pedikularkanülen (2a, 3a; 2b, 3b) besteht, die jeweils entweder auf dem oberen Wirbel Va oder auf dem unteren Wirbel Vb oder auf den beiden Wirbeln Va, Vb des Wirbelsegments eingerichtet sind, mindestens einen Führungsstift (4) mit gekrümmtem Profil, der in der Platte Pi, Ps des entsprechenden Wirbel Va, Vb durch die entsprechende Pedikularkanüle eines biegsamen kanülierten Bohrers (5) implantiert ist, **dadurch gekennzeichnet, dass** der Bohrer um den entsprechenden Führungsstift (4) geführt und in Hin- und Herbewegungen von einem kanülierten Antriebssystem (6) derart angetrieben ist, dass allmählich und auf gesteuerte Weise Fenster 11 in der unteren Platte Pi und der oberen Platte Ps jedes Wirbels Va, Vb geknabbert werden, und die Nukleingewebe einer Zwischenwirbelscheibe D zur Herstellung des Zwischenwirbelraums Es zerrissen werden, einer Injektionsvorrichtung (7), die an eine der geraden Pedikularkanülen (2a, 3a; 2b, 3b) zur Injektion des Transplantats (8) in den Zwischenwirbelraum Es, der derart hergestellt wird, angeschlossen ist, und Verschlüsse (9), die in die Löcher geschraubt werden, die nach dem Herausziehen der geraden Pedikularkanülen freigelassen werden, besteht

2. Vorrichtung nach Anspruch 1, **dadurch gekennzeichnet, dass** der biegsame kanülierte Bohrer (5) einen starren kanülierten Schaft (50) umfasst, der sich in einer biegsamen Längszone verlängert, die aus einem hohlen und biegsamen Torsionskabel (54) besteht, das an seinem freien Ende fest mit einem kanülierten Fräser (52), der geschliffene Profile aufweist, verbunden ist.

3. Vorrichtung nach Anspruch 2, **dadurch gekennzeichnet, dass** dem kanülierten Fräser (52) entgegengesetzt der starre Schaft (50) des kanülierten Bohrers (5) Verbindungsmittel umfasst, die eingerichtet sind, um eine Verbindung mit dem kanülierten Antriebssystem (6) zu bilden, die es erlaubt, den kanülierten Bohrer (5) in Hin- und Her- und Drehbewegungen anzutreiben, damit der kanülierte Fräser (52) allmählich und auf gesteuerte Weise Fenster F in die untere Platte Pi und die obere Platte Ps jedes Wirbels Va, Vb knabbert, und das Nukleingewebe zerreißt.

4. Vorrichtung nach Anspruch 1, **dadurch gekennzeichnet, dass** der gekrümmte Führungsstift (4) Verbindungsmittel umfasst, die sein Halten, die Steuerung seiner Positionierung und das Ausüben auf dieser Letzteren der Hin- und Herbewegungen erlauben.

5. Vorrichtung nach Anspruch 1, **dadurch gekennzeichnet, dass** die Verschlüsse (9) Pedikelschrauben (90) einer Wirbelbefestigungsvorrichtung sind, die das Anbringen und das Befestigen der Verbindungsschäfte (91) erlaubt.

6. Vorrichtung nach Anspruch 5, **dadurch gekennzeichnet, dass** jede Pedikelschraube (90) einen Verbindungsstecker umfasst, der mit einer Druckschraube (92) zum Stillstellen in Verschiebung und in Drehung eines Verbindungsschafts (91) zusammenwirkt.

## Claims

1. Device for placement and injection of an autologous or biological graft (6) into an intersomatic space Es previously arranged between two upper and lower vertebrae Va, Vb of a spinal segment in order to achieve an intersomatic fusion by totally percutaneous transpedicular approach, consisting of at least two straight pedicular cannulas (2a, 3a; 2h, 3b) arranged respectively either on the upper vertebra Va, or on the lower vertebra Vb or on both vertebrae Va, Vb of the spinal segment, at least one guide pin (4) with curved profile which is implanted in the endplate Pi, Ps of the corresponding vertebra Va, Vb through the corresponding pedicular cannula of a flexible cannulated drill bit (5), **characterized in that** the drill bit is guided around the corresponding guide pin (4) and driven in to-and-fro movements by a cannulated driving system (6), in such a way as to progressively, and in a controlled manner, nibble out windows F in said lower endplate Pi and upper endplate Ps of each vertebra Va, Vb and to shred the nucleic tissues of an intervertebral disc D in order to produce the intersomatic space Es, an injection device (7) connected to one of the straight pedicular cannulas (2a, 3a; 2b, 3b) in order to inject the graft (8) into the intersomatic space Es thus produced; and plugs (9) screwing into the holes left free after the removal of said straight pedicular cannulas.

2. Device according to claim 1, **characterized in that** the flexible cannulated drill bit (5) comprises a rigid cannulated rod (50) extended by a flexible longitudinal zone constituted by a hollow and flexible torsion cable (51) secured at its free end to a cannulated burr (52) having sharpened profiles.

3. Device according to claim 2, **characterized in that**, opposite the cannulated burr (52), the rigid rod (50) of the cannulated drill bit (5) comprises connection means arranged to form a connection with the cannulated driving system (6) making it possible to drive said cannulated drill bit (5) in to-and-fro and rotational movements so that the cannulated burr (52) progressively and in a controlled manner nibbles windows F in said lower endplate Pi and upper endplate Ps of each vertebra Va, Vb and to shred the nucleic tissues.

4. Device according to claim 1, **characterized in that** the curved guide pin (4) comprises connection means making it possible to held it, to control its positioning and to exert to-and-fro movements thereon.

5. Device according to claim 1, **characterized in that** the plugs (9) are pedicle screws (90) of a spinal fixation device the installation and attachment of connecting rods (91).

6. Device according to claim 5, **characterized in that** each pedicle screw (90) comprises a connecting connector (92) co-operating with a pressure screw (93) for the immobilization in translation and in rotation of a connecting rod (91).
